# EUROPEAN PATENT APPLICATION

(11) **EP 2 168 967 A1**
(43) Date of publication of application: **31.03.2010**
(21) Application number: 08787649.6
(22) Date of filing: 12.06.2008
(51) Int. Cl.: C07D 487/04, C07F 5/02

(54) **INTERMEDIATES FOR PREPARATION OF AN INHIBITOR OF PHOSPHODIESTERASE TYPE 5**

(30) Priority: 15.06.2007 ES 200701718
(71) Applicant: Galenicum Health, S.l., 08006 Barcelona (ES)
(72) Inventor: CANTOS LLOPART, Carme, E-08028 Barcelona (ES); COMELY, Alexander, Christian, E-08015 Barcelona (ES); RAFECAS JANÉ, Llorenç, E-43712 Llorenç del Penedès (Tarragona) (ES); TESSON, Nicolas, E-08007 Barcelona (ES)
(74) Representative: ZBM Patents
(86) International application number: PCT/ES2008/070115
(87) International publication number: WO 2008/152177

(57) **Abstract**

Boronic compounds of formula **II** and the process for their preparation, wherein Y₁ and Y₂ are selected from hydroxyl, (C₁-C₄)alcoxyl and phenoxyl optionally substituted with one or two radicals independently selected from (C₁-C₄)alkyl and (C₁-C₄)alcoxyl; or alternatively Y₁ and Y₂ together with the boron atom can form a cyclic system. Process for the preparation of Sildenafil by reaction of a intermediate of formula **II** with a intermediate of formula **III,** wherein X₃ is a leaving group and R₁ is selected from H and (C₁-C₄)alkyl.

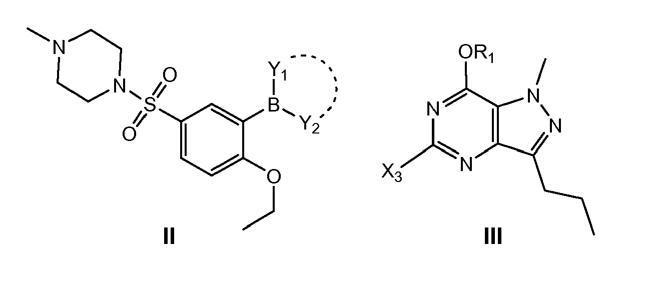

## Description

The present invention relates to a process for the preparation of Sildenafil, as well as to novel intermediates useful for its preparation, and to a preparation process of said intermediates.

### BACKGROUND OF THE INVENTION

Sildenafil is the generic name of the compound 5-[2-ethoxy-5-(4-methylpiperazin-1-ylsulfonyl)phenyl]-1-methyl-3-propyl-1,6-dihydro-7*H-*pyrazolo[4,3-*d*]pyrimidin-7-one, of formula: 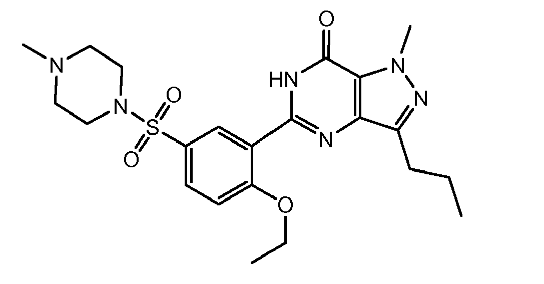 Sildenafil is a selective and potent phosphodiesterase type 5 (PDE5) inhibitor specific for cyclic guanosine monophosphate (cGMP) of the cavernous bodies, where the PDE5 is responsible for the cGMP breakdown. It was first described in the European Patent EP 463756 and its use for treating male erectile dysfunction has been shown (WO 94/28902).

Several preparation processes for Sildenafil have been described. Thus, for example, patents EP 463756 and EP 1077214 describe the coupling of a piperazine derivative in the last step of synthesis.

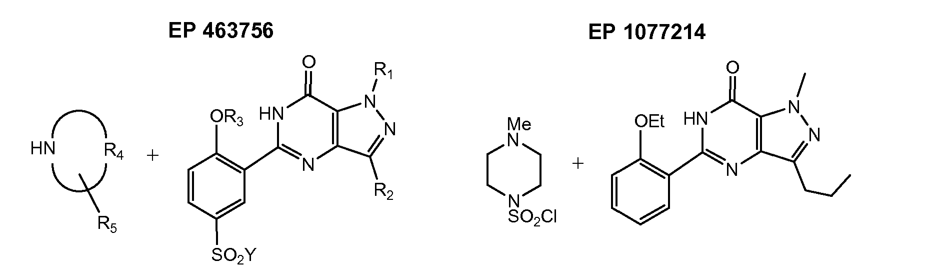

Moreover, documents EP 812845 and WO 01/98284 describe the cyclisation of the pyrazolopyrimidone ring as the last step of the Sildenafil synthesis.

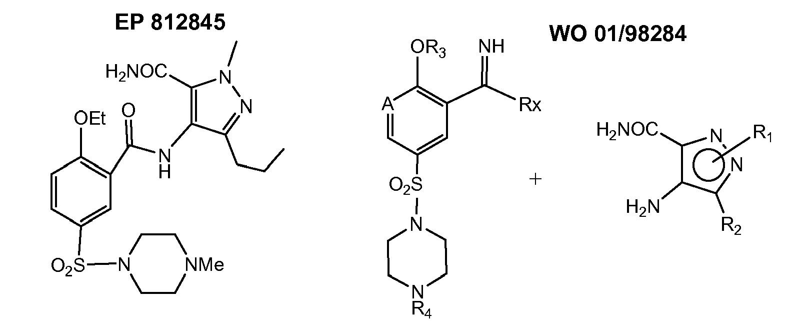

Moreover, other processes include a dehydrogenation reaction described in the application WO 01/98303, a reductive methylation described in the application WO 01/19827 and an internal cyclisation of the piperazine ring described in the patent US 6204383.

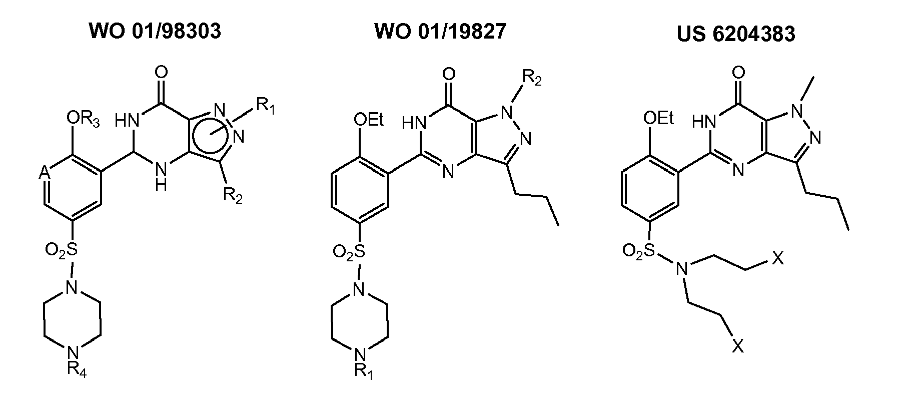

Hence, it is interesting to have alternative processes for the preparation of Sildenafil, especially if they are easy to industrialize.

### DESCRIPTION OF THE INVENTION

The inventors have found a new process for the preparation of Sildenafil starting from new boronic intermediates, proceeding with high yield and purity.

Thus, an aspect of the invention relates to a process for the preparation of a compound of formula **I,** or a tautomeric form thereof, or a pharmaceutically acceptable salt thereof, or a pharmaceutically acceptable solvate thereof, including a hydrate, 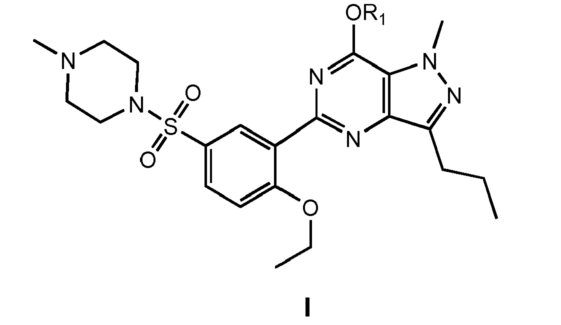 wherein R₁ is selected from H and (C₁-C₄)alkyl ; which comprises the coupling reaction of a compound of formula **II** 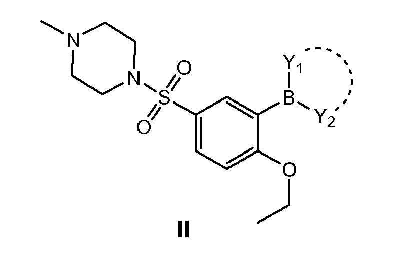 with a compound of formula **III** 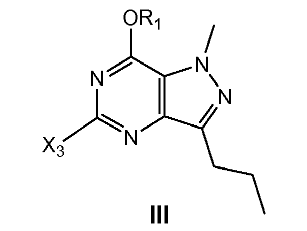 in a suitable solvent in the presence of a metal compound and a base, wherein:
Y₁ and Y₂ are selected from hydroxyl, (C₁-C₄)alcoxyl and phenoxyl optionally substituted with one or two radicals independently selected from (C₁-C₄)alkyl and (C₁-C₄)alcoxyl ; or alternatively Y₁ and Y₂ together with the boron atom may form one of the following cyclic systems: 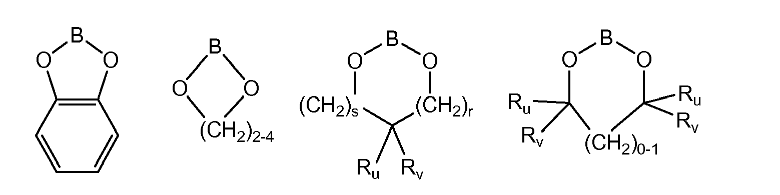 wherein r and s independently represent a value between 0 and 4, provided that r and s cannot be simultaneously 0; and Rᵤ and Rᵥ are independently selected from H, (C₁-C₄)alkyl and phenyl optionally substituted with one or two radicals independently selected from (C₁-C₄)alkyl and (C₁-C₄)alcoxyl;
X₃ is a leaving group; and R₁ has the same meaning as in the compound of formula **I;**
and, optionally, the compound of formula **I** whereinR₁ is (C₁-C₄)alkyl undergoes a hydrolysis reaction to give the compound of formula **I** wherein R₁ is H; and, optionally, the compound of formula **I** is converted into a pharmaceutically acceptable salt thereof by treatment with the corresponding acid, or a salt of the compound of formula **I** may be converted into another salt of the compound of formula **I** by ion exchange, or the compound of formula **I** is converted into a pharmaceutically acceptable solvate thereof, including a hydrate, by crystallization/precipitation in a suitable solvents system.

Another aspect of the invention relates to a compound of formula **I,** or a pharmaceutically acceptable salt thereof, or a pharmaceutically acceptable solvate thereof, including a hydrate, 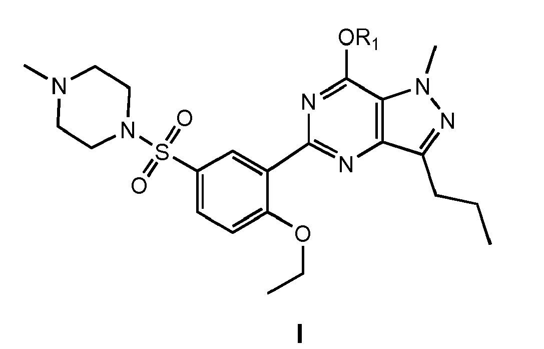 wherein R₁ represents (C₂-C₄)alkyl.

Another aspect of the invention relates to an intermediate of formula **II** 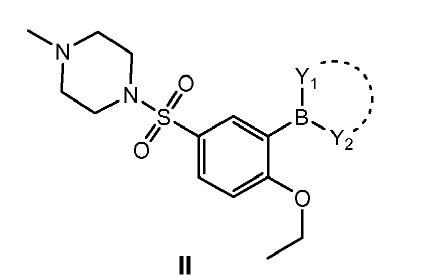 wherein Y₁ and Y₂ have the meaning described above.

Another aspect of the invention relates to a preparation process of a compound of formula **II**, which comprises the reaction of a compound of formula **IV**, 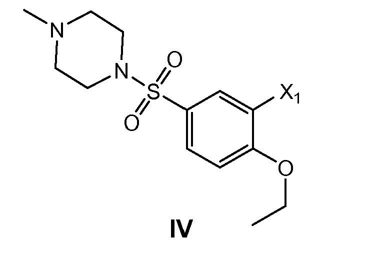 with a metal compound in a suitable solvent, and subsequently, with a boronic derivative of formula **XI**, 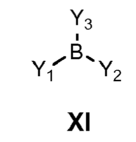 wherein X₁ is a leaving group; and Y₁, Y₂ and Y₃ represent (C₁-C₄)alcoxyl ;
and, optionally, the obtained compound of formula **II**, wherein Y₁ and Y₂ represent hydroxyl, may be converted into another compound of formula **II** wherein Y₁ and Y₂ are selected from (C₁-C₄)alcoxyl and phenoxyl optionally substituted with one or two radicals independently selected from (C₁-C₄)alkyl and (C₁-C₄)alcoxyl ;
by reaction with an alcohol of formula R₂OH in a suitable solvent, wherein R₂ is selected from (C₁-C₄)alkyl and phenyl optionally substituted with one or two radicals independently selected from (C₁-C₄)alkyl and (C₁-C₄)alcoxyl ; or optionally, the obtained compound of formula **II**, wherein Y₁ and Y₂ represent hydroxyl, may be converted into another compound of formula **II** wherein Y₁ and Y₂ together with the boron atom may form one of the following cyclic systems: 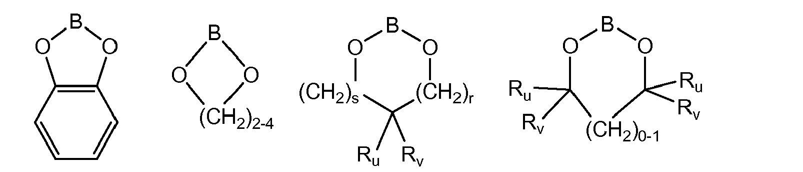 by reaction with a diol of formula: 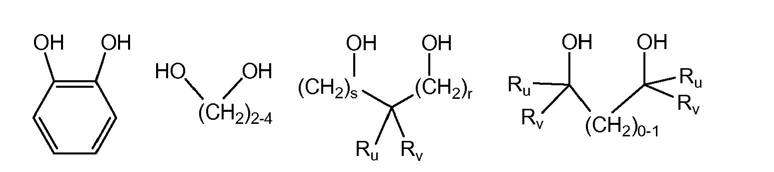 in a suitable solvent, wherein r, s, Rᵤ and Rᵥ have the meaning described above.

The compounds of formula **III,** as well as their preparation process, are new and are part of the invention. Thus, the present invention also relates to the intermediates of formula **III** 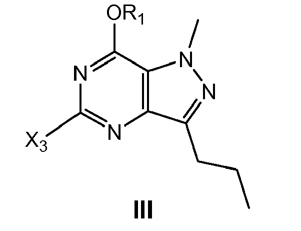 wherein X₃ is a leaving group, and R₁ is selected from H and (C₁-C₄)alkyl .

Likewise, the present invention also relates to a preparation process of a compound of formula **III,** which comprises the reaction of a compound of formula **VIII** 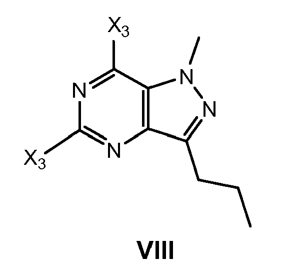 wherein X₃ is a leaving group, in a suitable solvent and in the presence of a base.

The term (C₁-C₄)alkyl, used throughout the description, means a radical of a linear or branched saturated hydrocarbon chain containing from 1 to 4 carbon atoms. Examples include methyl, ethyl, propyl, isopropyl, butyl, isobutyl, sec-butyl and *tert*-butyl groups.

The term (C₁-C₄)alcoxyl means a radical of oxygen of formula -O(C₁-C₄)alkyl, wherein (C₁-C₄)alkyl has the meaning described above. Examples include methoxyl, ethoxyl, propoxyl, isopropoxyl, butoxyl, isobutoxyl, sec-butoxyl and *tert*-butoxyl groups.

The term phenoxyl means a radical of oxygen of formula -OPh and may be optionally substituted as indicated.

The term halogen includes the fluorine, chlorine, bromine and iodine atoms.

As it will be apparent for a person skilled in the art, some compounds may exist in various tautomeric forms, all of them included in the present invention. Thus, for example, a compound of formula **I** or formula **III** wherein R₁ is H, i.e. a compound of formula **Ia** or a compound of formula **IIIa** respectively 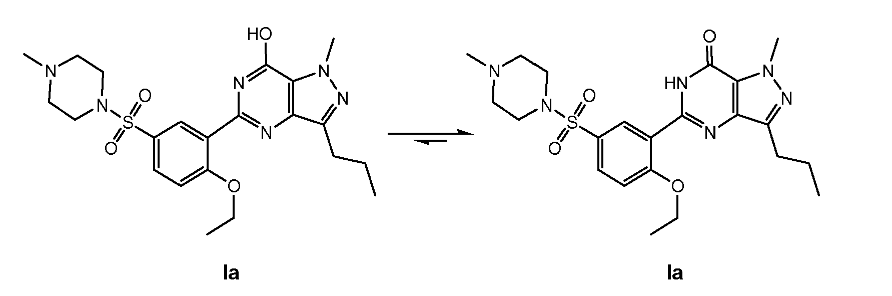 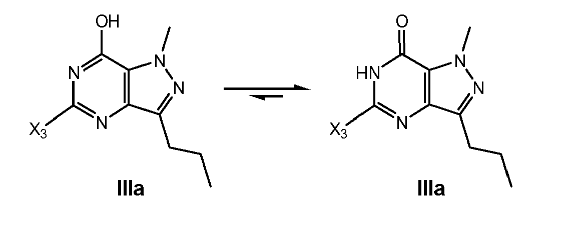 may exist in enolic or ketonic form. Both forms are equivalent for the purpose of the invention and are indistinctly mentioned throughout the description and claims, taking in account that every time that one of them is mentioned, its corresponding tautomeric form it is also included.

As it has been described above, the preparation process of a compound of formula **I** comprises a coupling reaction of a compound of formula **II** with a compound of formula **III**: 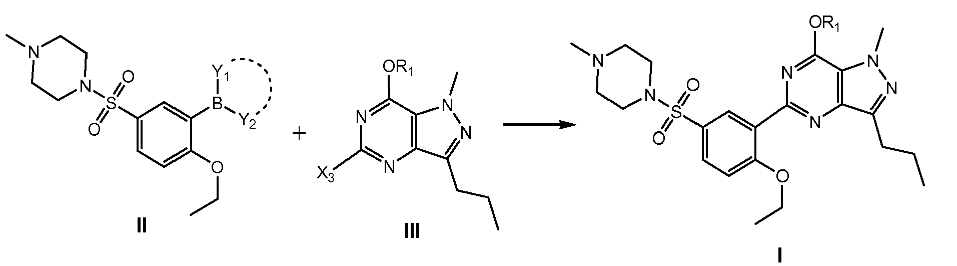 This reaction is known as a Suzuki coupling reaction. It is carried out in the presence of a suitable solvents system and a metal compound. The best reaction conditions (temperature, solvent and others) may be varied depending on the starting compounds and may be readily determined by a person skilled in the art.

Preferably the metal compound is selected from palladium, nickel, a metal salt and a metal complex. More preferably the metal compound is selected from Pd(PPh₃)₄, PdCl₂(dppf) and Pd(OAc)₂/PPh₃.

Preferably, the solvents system is selected from water, an organic solvent selected from the aromatic (C₆-C₈)hydrocarbons, a polar aprotic solvent and aliphatic (C₂-C₈)ethers; and mixtures of water and one or more organic solvents of the aforementioned. More preferably, the solvents system consists of dimethoxyethane.

Preferably, the base is selected from an alkali metal carbonate and an alkali metal phosphate. More preferably the base is selected from sodium carbonate, potassium carbonate and potassium phosphate.

Preferably the reaction is carried out at a temperature comprised between room temperature and the reflux temperature of the used solvent. More preferably the reaction is carried out at reflux.

A preferred embodiment relates to a preparation process of a compound of formula I, or a pharmaceutically acceptable salt thereof, or a pharmaceutically acceptable solvate thereof, including a hydrate, wherein Y₁ and Y₂ are selected from hydroxyl, (C₁-C₄)alcoxyl and phenoxyl optionally substituted with one or two radicals independently selected from (C₁-C₄)alkyl and (C₁-C₄)alcoxyl. In a more preferred embodiment, Y₁ and Y₂ represent hydroxyl.

Another preferred embodiment relates to the above preparation process wherein X₃ is selected from Cl, Br, I and trifluoromethanesulfonyloxyl. In another more preferred embodiment X₃ is selected from Cl, Br and I. In another even more preferred embodiment X₃ is Cl.

By way of example, in a particular embodiment, when in the preparation process of a compound of formula **I** an intermediate of formula **III** wherein R₁ is H is used; the compound of formula **I** obtained is Sildenafil (**Ia**); which may be converted into a pharmaceutically acceptable salt or a pharmaceutically acceptable solvate, including a hydrate.

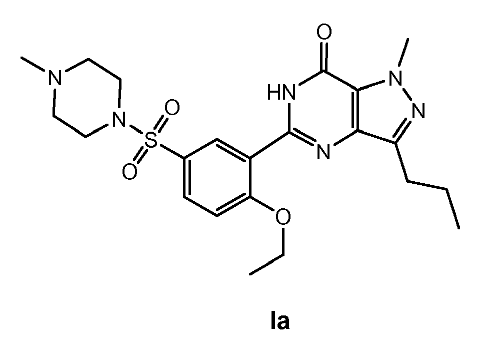

The formation of said salts may be carried out by treatment of the compound of formula **I** with a sufficient amount of the desired acid. Both organic and inorganic acid salts may be used. Examples of inorganic acids include hydrochloric acid, hydrobromic acid, hydroiodic acid, nitric acid, perchloric acid, sulfuric acid or phosphoric acid. Examples of organic acids include methanesulfonic acid, trifluoromethanesulfonic acid, ethanesulfonic acid, benzenesulfonic acid, *p*-toluenesulfonic acid, fumaric acid, citric acid, oxalic acid, acetic acid and maleic acid, among others. There is no limitation in the nature of said salts, provided that when they are used for therapeutic purposes they are pharmaceutically acceptable. In turn, the salts of the compounds of formula **I** may be transformed into other salts of compounds of formula **I** by ion exchange, for example, by an ion exchange resin or by reaction with another salt. A preferred salt of Sildenafil is the citrate.

Moreover, when an intermediate of formula **III** wherein R₁ is (C₁-C₄)alkyl is used, the obtained compound of formula **I** is **Ib**: 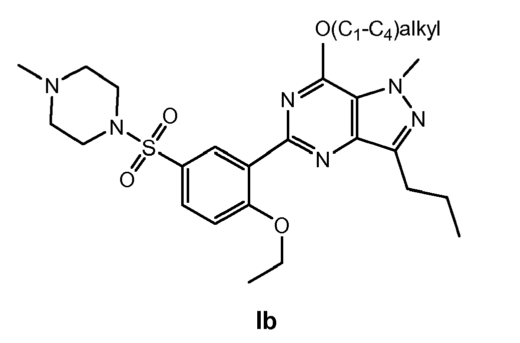 which may be converted into Sildenafil by a further hydrolysis step; or the compound **Ib** may be converted into a pharmaceutically acceptable salt thereof or a pharmaceutically acceptable solvate thereof, including a hydrate, under the aforementioned conditions for Sildenafil.

The hydrolysis reaction may be carried out in acid medium, for example in HCl in a suitable solvent, such as for example methanol, and at a suitable temperature, preferably by heating, or with a Lewis acid, such as trimethylsilyl chloride in the presence of Nal, in a suitable solvent, such as for example acetonitrile.

The present invention also relates to the compounds of formula **I** wherein R₁ represents (C₂-C₄)alkyl. In a preferred embodiment the compound of formula I is 7-ethoxy-5-[2-ethoxy-5-(4-methylpiperazin-1-ylsulfonyl)phenyl]-1-methyl-3-propyl-1*H*-pyrazolo[4,3-*d*]pyrimidine.

As mentioned above, the intermediates of formula **II** are part of the invention.

In a preferred embodiment, Y₁ and Y₂ are selected from hydroxyl, (C₁-C₄)alcoxyl and phenoxyl optionally substituted with one or two radicals independently selected from (C₁-C₄)alkyl and (C₁-C₄)alcoxyl. In a more preferred embodiment, Y₁ and Y₂ represent hydroxyl. In the most preferred embodiment the compound of formula **II** is [2-ethoxy-5-(4-methylpiperazin-1-ylsulfonyl)]phenylboronic acid.

The compounds of formula **II** may be prepared by reaction of an intermediate of formula **IV** with a metal compound, such as for example *ⁱ*PrMgCl·LiBr, and subsequently with a boronic derivative of formula **XI**:

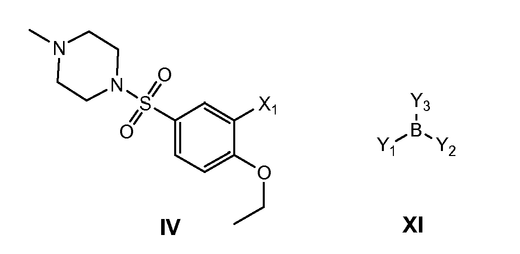

In a preferred embodiment, X₁ is selected from Br and I. In a more preferred embodiment, X₁ is Br. In another preferred embodiment, Y₁, Y₂ and Y₃ represent ethoxyl.

This reaction is carried out in a suitable solvent such as for example tetrahydrofuran and at a suitable temperature, preferably at room temperature.

After an aqueous treatment, a boronic acid of formula **II** is obtained, wherein Y₁ and Y₂ represent hydroxyl. As mentioned hereinbefore, this compound may be optionally converted into an open or cyclic boronic ester of formula **II**. This reaction is carried out in the presence of an alcohol to give an open boronic ester, or a diol to give a cyclic boronic ester; in a suitable solvent, such as for example tetrahydrofuran or toluene, optionally using a dehydrating system and at a suitable temperature, optionally by heating.

The intermediates of formula **IV** may be obtained by means of the following synthetic pathway:

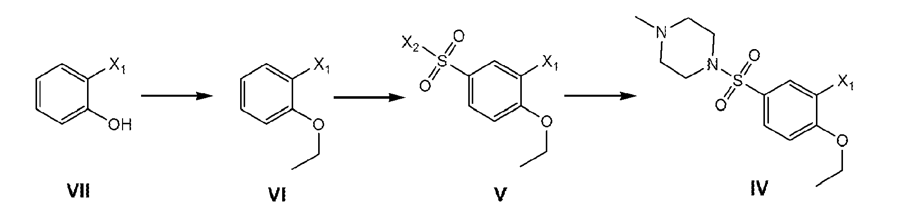

In a first step a halophenol of formula **VII** is alkylated, with an alkylating agent of formula CH₃CH₂X to give an ether of formula **VI,** wherein X and X₁ represent independently a leaving group, preferably X and X₁ are Br. This reaction is carried out in the presence of a base, such as for example potassium carbonate, optionally in the presence of catalytic amounts of Nal, in a suitable solvent, such as for example acetone and at a suitable temperature, preferably at room temperature.

Subsequently, sulfonylation of a compound of formula **VI** with a sulfonating agent, such as for example chlorosulfonic acid, gives rise to a compound of formula **V,** wherein X₂ represents halogen, preferably chlorine. This reaction is carried out in a suitable solvent, such as for example dichloromethane and at a suitable temperature, preferably at room temperature.

The substitution of the X₂ group in the compound of formula **V** by *N-*methylpiperazine gives rise to the compound of formula **IV**. This reaction is carried out in the presence of a base, such as for example triethylamine, in a suitable solvent, such as for example acetone and at a suitable temperature, preferably room temperature.

As mentioned hereinbefore, the intermediates of formula **III** are also considered to be part of the invention:

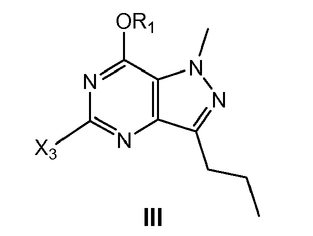

In a preferred embodiment, X₃ is selected from Cl, Br and I. In yet another more preferred embodiment X₃ is Cl. In another preferred embodiment, R₁ is methyl or ethyl. In another preferred embodiment, R₁ is H. In the most preferred embodiment the compound of formula **III** is selected from 5-chloro-1-methyl-3-propyl-1*H*-pyrazolo[4,3-*d*]pyrimidin-7(6H)-one, 5-chloro-7-methoxy-1-methyl-3-propyl-1*H*-pyrazolo[4,3-*d*]pyrimidine and 5-chloro-7-ethoxy-1-methyl-3-propyl-1*H*-pyrazolo[4,3-*d*]pyrimidine.

The compounds of formula **III** of the present invention may be prepared by reaction of a compound of formula **VIII** 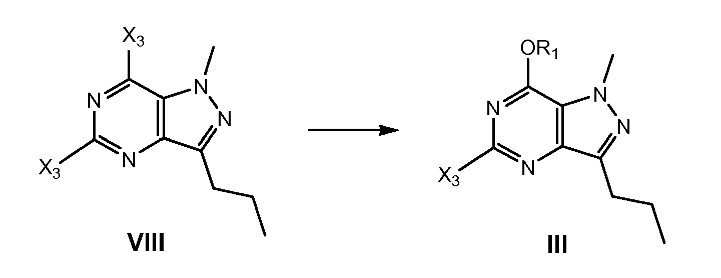 in a suitable solvent and in the presence of a hydroxide of formula MOH, wherein M is a cation, preferably an alkali or alkaline-earth metal, for example KOH or NaOH, to give a compound of formula **III** wherein R₁ is H; or in the presence of an alkoxide of formula MO(C₁-C₄)alkyl, wherein M is a cation, preferably an alkali or alkaline-earth metal, for example sodium methoxide or sodium ethoxide, to give a compound of formula **III** wherein R₁ is (C₁-C₄)alkyl. The reaction takes place in a suitable solvent, such as for example tetrahydrofuran when KOH or NaOH are used, methanol if sodium methoxide is the used base or ethanol if the base is sodium ethoxide, and at a suitable temperature, preferably room temperature.

In a preferred embodiment, in the compound of formula **VIII**, X₃ is selected from Cl, Br and I. In another more preferred embodiment X₃ is Cl.
The compounds of formula **VIII** may be obtained by processes similar to those described in the application WO 2006/046135 (example 43 corresponding to a compound of formula **VIII** wherein X₃ is Cl, page 114, lines 1-7).

An advantage of the present invention is that it relates to a process of convergent synthesis, proceeding with high yields. Likewise, the used starting products are inexpensive and available on an industrial scale.

Throughout the description and the claims the word "comprises" and its variants are not intended to exclude other technique characteristics, additives, components or steps.

Other objects, advantages and characteristics of the invention will be apparent for the person skilled in the art partly from the description and partly from the implementation of the invention. The following examples are provided by way of illustration only and are not intended to limit the present invention.

### EXAMPLES

### Intermediate VI.1 (X₁ = Br): 1-bromo-2-ethoxybenzene

To a suspension of 10.82 g (72 mmol) of Nal and 79.9 g (578 mmol) of K₂CO₃ in 150 mL of acetone, 25 g (144.5 mmol) of 1-bromophenol and, then, 21.6 mL (289 mmol) of bromoethane were added. The reaction was kept stirring at reflux overnight. The reaction mixture was cooled to room temperature and was evaporated to dryness in a rotary evaporator. The residue was suspended in ethyl ether and the solid in suspension was filtered and washed with ethyl ether. The filtrate was dried over anhydrous sodium sulfate and evaporated to dryness under vacuum to obtain 27.7 g (95% yield) of 1-bromo-2-ethoxybenzene as a colorless oil.
¹H NMR (CDCl₃, 400 MHz) δ (ppm): 7.53 (dd, *J* = 1.6 Hz, *J* = 7.8 Hz, 1H) ; 7.24 (ddd, *J* = 1.6 Hz, *J* = 7.6 Hz, *J* = 8.4 Hz, 1H); 6.89 (dd, *J* = 1.4 Hz, *J* = 8.4 Hz, 1H); 6.82 (ddd, *J* = 1.4 Hz, *J* = 7.6 Hz, *J* = 7.6 Hz, 1H); 4.10 (q, *J* = 6.9 Hz, 2H) ; 1.47 (t, *J* = 6.9 Hz, 3H).

### Intermediate V.1 (X₁ = Br, X₂ = Cl): 1-bromo-2-ethoxybenzene-1-sulfonyl chloride

To a solution of 9 g (45 mmol) of 1-bromo-2-ethoxybenzene (intermediate **VI.1)** in 220 mL of dichloromethane, a solution of 9 mL (135 mmol) of chlorosulfonic acid in 16 mL of dichloromethane was added for one hour at -5°C. Then it was stirred for an additional 1 hour, at room temperature. The reaction mixture was carefully poured over about 500 g of crushed ice and the resulting phases were allowed to decant and were separated. The organic phase was washed, successively, with 220 mL of a 5% Na₂CO₃ aqueous solution, 220 mL of NaHCO₃ saturated aqueous solution, 220 mL of water and 220 mL of brine. Then, it was dried over anhydrous sodium sulfate and evaporated to dryness to obtain 11.91 g (89% yield) of 1-bromo-2-ethoxybenzene-1-sulfonyl chloride as a yellow oil.
¹H NMR (CDCl₃, 200 MHz) δ (ppm): 8.21 (d, *J* = 2.2 Hz, 1H); 7.96 (dd, *J* = 2.2 Hz, *J* = 9.2 Hz, 1H) ; 7.01 (d, *J* = 9.2 Hz, 1H); 4.24 (q, *J* = 7.0 Hz, 2H) ; 1.55 (t, *J* = 7.0 Hz, 3H).

### Intermediate IV.1 (X₁ = Br): 1-(3-bromo-4-ethoxybenzenesulfonyl)-4-methyl-piperazine

To a solution of 11.9 g (40 mmol) of 1-bromo-2-ethoxybenzene-1-sulfonyl chloride (intermediate V.1) in 33 mL of acetone, and under nitrogen atmosphere, 4.85 mL (44 mmol) of N-methylpiperazine and 6.1 mL (44 mmol) of triethylamine were successively added. Then, the reaction mixture was stirred at room temperature for 3 hours and was evaporated under vacuum to dryness. The dry residue was dissolved in 60 mL of dichloromethane and the resulting solution was washed, first 3 times with 50 mL of 1 M sodium hydroxide aqueous solution, and then, 3 times with 50 mL of water. The organic phase was dried over anhydrous sodium sulfate and evaporated under vacuum to dryness. Thus, 13.5 g (94% yield) of the compound **IV.1** as a yellow solid were obtained.
¹H NMR (CDCl₃, 200 MHz) δ (ppm): 7.93 (d, *J* = 2.2 Hz, 1H) ; 7.65 (dd, *J* = 2.2 Hz, *J* = 8.8 Hz, 1H) ; 6.95 (d, *J* = 8.8 Hz, 1H); 4.17 (q, *J* = 7.0 Hz, 2H) ; 3.11-2.99 (m, 4H) ; 2.58-2.43 (m, 4H) ; 2.29 (s, 3H) ; 1.51 (t, *J* = 7.0 Hz, 3H).

### Intermediate II.1 (Y₁, Y₂ = OH): [2-ethoxy-5-(4-methylpiperazin-1-ylsulfonyl)]phenylboronic acid

To a solution of 500 mg (1.4 mmol) of 1-(3-bromo-4-ethoxybenzenesulfonyl)-4-methylpiperazine (intermediate **IV.1)** in 3.2 mL of anhydrous tetrahydrofuran (THF) solution and under argon atmosphere, 1.03 mL of a 2 M solution of isopropyl magnesium chloride with lithium bromide 1:1 complex (*ⁱ*PrMgCl.LiBr) in THF was added dropwise and at a temperature of 0°C. The reaction mixture was kept stirring at room temperature for 4 hours. Then, it was cooled again to 0°C and 0.59 mL (3.45 mmol) of triethyl borate (B(OEt)₃) were added. The reaction mixture was then kept stirring at room temperature overnight. The next day, 6 mL of ammonium chloride saturated solution in water were added to the reaction mixture and the THF present in the medium was distilled off under vacuum. The resulting aqueous phase was extracted 3 times with 5 mL of ethyl acetate and the resulting organic phases were combined and evaporated to dryness. The residue was dissolved in 10 mL of 1 M sodium hydroxide aqueous solution and the resulting solution was washed 2 times with 6 mL of dichloromethane. Then the aqueous phase was acidified with a 10% aqueous solution of hydrochloric acid to reach pH 1 and then it was basified to pH 8 with 1 M sodium hydroxide. The resulting aqueous solution was extracted 3 times with 20 mL of ethyl acetate and the combined organic phases dried over anhydrous sodium sulfate and evaporated to dryness under vacuum. 280 mg (63% yield) of the compound **II.1** as a pale yellow solid were obtained.
¹H NMR (CDCl₃, 400 MHz) δ (ppm): 8.19 (d, *J* = 2.5 Hz, 1H) ; 7.81 (dd, *J* = 2.5 Hz, *J* = 8.8 Hz, 1H) ; 6.98 (d, *J* = 8.8 Hz, 1H); 4.21 (q, *J* = 7.0 Hz, 2H) ; 3.20-2.98 (m, 4H) ; 2.66-2.44 (m, 4H) ; 2.30 (s, 3H) ; 1.52 (t, *J* = 7.0 Hz, 3H).

### Intermediate III.1 (X₃ = Cl, R₁ = H): 5-chloro-1-methyl-3-propyl-1H-pyrazolo[4,3-d]pyrimidin-7(6H)-one

To a solution of 400 mg (1.63 mmol) of 5,7-dichloro-1-methyl-3-propyl-1*H-*pyrazolo[4,3-*d*]pyrimidine (obtained according to example 43 of the application WO 2006/046135, page 114, lines 1-7) in 3.4 mL of THF, was added a solution of 415 mg (6.28 mmol) of 85% sodium hydroxide in 3.1 mL of water. The reaction mixture was stirred at room temperature for 3 hours. Then glacial acetic acid was added until reaching pH 7. The reaction mixture was extracted 3 times with 10mL ethyl acetate each time and the resulting organic phases were dried over anhydrous sodium sulphate and evaporated to dryness under vacuum to give 355 mg (96% yield) of the compound **III.1** as a white solid.
¹H NMR (CDCl₃, 400 MHz) δ(ppm): 4.24 (s, 3H) ; 2.84 (dd, *J* = 7.3 Hz, *J* = 7.8 Hz, 2H) ; 1.84-1.72 (m, 2H) ; 1.00 (t, *J* = 7.3 Hz, 3H).

### Intermediate III.2 (X₃ = Cl, R₁ = methyl): 5-chloro-7-methoxy-1-methyl-3-propyl-1H-pyrazolo[4,3-d]pyrimidine

To a solution of 1 g (4.1 mmol) of 5,7-dichloro-1-methyl-3-propyl-1*H-*pyrazolo[4,3-*d*]pyrimidine (obtained according to example 43 of the application WO 2006/046135, page 114, lines 1-7) in 1.4 mL of anhydrous methanol at 0°C, a solution of 0.22 g (4.1 mmol) of sodium methoxide in 2.8 mL of anhydrous methanol was added by cannulation. The reaction mixture was left to reach room temperature overnight.
The resulting suspension was concentrated under reduced pressure, the residue was dissolved in 25 mL of diethyl ether and was filtered to remove the sodium chloride produced during the reaction. The salts were washed with an excess of diethyl ether and the filtrate was concentrated to dryness under reduced pressure to give 0.85 g (87% yield) of the compound **III.2** as a white solid.
¹H NMR (CDCl₃, 200 MHz) δ(ppm): 4.19 (s, 3H) ; 2.92 (dd, *J* = 7.3 Hz, *J* = 8.0 Hz, 2H) ; 1.93-1.71 (m, 2H) ; 0.99 (t, *J* = 7.3 Hz, 3H).

### Intermediate III.3 (X₃ = Cl, R₁ = ethyl): 5-chloro-7-ethoxy-1-methyl-3-propyl-1H-pyrazolo[4,3-d]pyrimidine

To a solution of 0.2 g (0.81 mmol) of 5,7-dichloro-1-methyl-3-propyl-1*H-*pyrazolo[4,3-*d*]pyrimidine (obtained according to example 43 of the application WO 2006/046135, page 114, lines 1-7) in 0.5 mL of anhydrous ethanol at 0°C, a solution of 55 mg (0.81 mmol) of sodium ethoxide in 0.8 mL of anhydrous ethanol was added by cannulation. The reaction mixture was left to reach room temperature overnight. As the reaction was not complete, 20 mg (0.29 mmol) of sodium ethoxide in 0.2 mL of anhydrous ethanol at 0°C were added and the reaction mixture was allowed to reach room temperature with stirring for 2 hours.
The resulting suspension was concentrated under vacuum, the residue was dissolved in 25 mL of diethyl ether and was filtered to remove the sodium chloride produced during the reaction. The salts were washed with an excess of diethyl ether and the filtrate was concentrated to dryness under reduced pressure to give 0.2 g (97% yield) of the compound **III.3** as a white solid.
¹H NMR (CDCl₃, 200 MHz) δ(ppm): 4.66 (q, *J* = 7.3 Hz, 2H) ; 4.18 (s, 3H) ; 2.92 (dd, *J* = 7.3 Hz, *J* = 8.0 Hz, 2H) ; 1.94-1.70 (m, 2H) ; 1.52 (t, *J* = 7.3 Hz, 3H) ; 1.00 (t, *J* = 7.3 Hz, 3H).

### Compounds of formula I

### General process A

To a Schlenk tube containing a solution of intermediate **II** (1 equiv.) and intermediate **III** (1 equiv.) in dimethoxyethane (DME) in a proportion of 20 mL of DME per gram of intermediate **III**, a solution of sodium carbonate (3.1 equiv.) in the minimum volume of water, 0.1 equiv. of palladium acetate (II) and 0.4 equiv. of triphenylphosphine were successively added. The reaction mixture was purged with argon and heated to reflux overnight.
The next day the reaction mixture was evaporated to dryness under vacuum. The residue was dissolved in dichloromethane and washed two times with water. The organic phase was evaporated to dryness under vacuum and was dissolved in toluene. The toluene phase was extracted two times with 1 M hydrochloric acid. The resulting aqueous phase was neutralised with 25% aqueous sodium hydroxide until reaching pH 14 and was extracted two times with ethyl acetate. The combined organic phases were dried over anhydrous sodium sulphate and evaporated to dryness under vacuum to give the product desired.

### Compound Ib.1 (R₁ = methyl): 5-[2-ethoxy-5-(4-methylpiperazin-1-ylsulfonyl)phenyl]-7-methoxy-1-methyl-3-propyl-1H-pyrazolo[4,3-d]pyrimidine

Following the general process A: starting from a solution of 245 mg (0.76 mmol) of [2-ethoxy-5-(4-methylpiperazin-1-ylsulphonyl)]phenylboronic acid (intermediate **II.1)** and 182 mg (0.76 mmol) of 5 chloro-7-methoxy-1-methyl-3-propyl-1*H*-pyrazolo[4,3-*d*]pyrimidine (intermediate **III.2)** in 3.7 mL of dimethoxyethane, 250 mg (2.35 mmol) of sodium carbonate, 17 mg (0.076 mmol) of palladium acetate and 80 mg (0.3 mmol) of triphenylphosphine, the reaction gave 276 mg (75% yield) of the compound **Ib.1** as a white solid.
¹H NMR (CDCl₃, 400 MHz) δ(ppm): 8.20 (d, *J* = 2.3 Hz, 1H) ; 7.75 (dd, *J* = 2.3 Hz, *J* = 8.7 Hz, 1H); 7.09 (d, *J* = 8.7 Hz, 1H); 4.23 (s, 3H) ; 4.19 (s, 3H) ; 4.17 (q, *J* = 7.0 Hz, 2H) ; 3.25-3.05 (m, 4H) ; 3.00 (dd, *J* = 7.5 Hz, *J* = 7.8 Hz, 2H) ; 2.69-2.45 (m, 4H) ; 2.33 (s, 3H) ; 1.96-1.84 (m, 2H); 1.42 (t, *J* = 7.0 Hz, 3H) ; 1.02 (t, *J* = 7.5 Hz, 3H).

### Compound Ib.2 (R₁ = ethyl): 7-ethoxy-5-[2-ethoxy-5-(4-methylpiperazin-1-ylsulfonyl)phenyl]-1-methyl-3-propyl-1H-pyrazolo[4,3-d]pyrimidine.

Following the general process A: starting from a solution of 200 mg (0.62 mmol) of [2-ethoxy-5-(4-methylpiperazin-1-ylsulphonyl)]phenylboronic acid (intermediate **II.1)** and 158 mg (0.62 mmol) of 5-chloro-7-ethoxy-1-methyl-3-propyl-1*H*-pyrazolo[4,3-*d*]pyrimidine (intermediate **III.3)** in 3.2 mL of dimethoxyethane, 204 mg (1.92 mmol) of sodium carbonate, 14 mg (0.062 mmol) of palladium acetate and 65 mg (0.25 mmol) of triphenylphosphine, the reaction gave 205 mg (66% yield) of the compound **Ib.1** as a yellow solid.
¹H NMR (CDCl₃, 400 MHz) δ(ppm): 8.17 (d, *J* = 2.5 Hz, 1H) ; 7.75 (dd, *J* = 2.5 Hz, *J* = 8.8 Hz, 1H) ; 7.08 (d, *J* = 8.8 Hz, 1H); 4.67 (q, *J* = 7.0 Hz, 2H) ; 4.24 (s, 3H) ; 4.17 (q, *J* = 7.0 Hz, 2H) ; 3.19-3.03 (m, 4H) ; 3.00 (dd, *J* = 7.4 Hz, *J* = 7.8 Hz, 2H) ; 2.60-2.42 (m, 4H) ; 2.30 (s, 3H) ; 1.97-1.83 (m, 2H) ; 1.51 (t, *J* = 7.0 Hz, 3H) ; 1.41 (t, *J* = 7.0 Hz, 3H) ; 1.02 (t, *J* = 7.4 Hz, 3H).

### Compound Ia (R₁ = H): 5-[2-ethoxy-5-(4-methylpiperazin-1-ylsulfonyl)phenyl]-1-methyl-3-propyl-1,6-dihydro-7H-pyrazolo[4,3-d]pyrimidin-7-one

Following the general process A: starting from a solution of 245 mg (0.76 mmol) of [2-ethoxy-5-(4-methylpiperazin-1-ylsulphonyl)]phenylboronic acid (intermediate **II.1)** and 172 mg (0.76 mmol) of 5-chloro-1-methyl-3-propyl-1*H-*pyrazolo[4,3-*d*]pyrimidine (intermediate **III.1)** in 3.6 mL of dimethoxyethane, 250 mg (2.35 mmol) of sodium carbonate, 17 mg (0.076 mmol) of palladium acetate and 79 mg (0.3 mmol) of triphenylphosphine, the reaction gave 215 mg (75% yield) of the raw compound **Ia** as a yellow solid. The solid was purified by chromatography in a silica gel column and eluted with a mixture of ethanol and 1% triethylamine to give 120 mg of impure compound Ia. The product was recrystallised in methanol to purify it.
¹H NMR (CDCl₃, 400 MHz) δ(ppm): 10.80 (s, N*H*) ; 8.84 (d, *J* = 2.3 Hz, 1H) ; 7.84 (dd, *J* = 2.3 Hz, *J* = 8.8 Hz, 1H) ; 7.15 (d, *J* = 8.8 Hz, 1H); 4.38 (q, *J* = 7.0 Hz, 2H) ; 4.28 (s, 3H) ; 3.23-3.06 (m, 4H) ; 2.93 (dd, *J* = 7.4 Hz, *J* = 7.8 Hz, 2H) ; 2.65-2.48 (m, 4H) ; 2.30 (s, 3H) ; 1.92-1.80 (m, 2H) ; 1.65 (t, *J* = 7.0 Hz, 3H) ; 1.02 (t, *J* = 7.4 Hz, 3H).

### General process B

To a solution of compound **Ib** (R₁ = (C₁-C₄)alkyl) in methanol (8.15 vol.), 6N hydrochloric acid (6.7 vol.) was added and the reaction mixture was heated to 75°C for 2 to 4 hours. Then the reaction mixture was allowed to reach room temperature, was diluted in water and a 25% aqueous solution of sodium hydroxide was added to reach pH 14. The aqueous phase was extracted two times with ethyl acetate and the resulting organic phases were dried over anhydrous sodium sulphate and evaporated to dryness under vacuum to give compound **Ia** as the raw product.

Following the general process **B:** starting from 270 mg (0.55 mmol) of 5-[2-ethoxy-5-(4-methylpiperazin-1-ylsulfonyl)phenyl]-7-methoxy-1-methyl-3-propyl-1*H*-pyrazolo[4,3-*d*]pyrimidine (compound **Ib.1),** 2.2 mL of methanol and 1.8 mL of 6 N hydrochloric acid, the reaction gave, after 4 hours, 215 mg (82% yield) of the compound **Ia** as a raw product.

Following the general process B: starting from 80 mg (0.16 mmol) of 7-ethoxy-5-[2-ethoxy-5-(4-methylpiperazin-1-ylsulfonyl)phenyl]-1-methyl-3-propyl-1*H-*pyrazolo[4,3-*d*]pyrimidine (compound **Ib.2**), 0.65 mL of methanol and 0.54 mL of 6 **N** hydrochloric acid, the reaction gave, after 4 hours and a half, 69 mg (91 % yield) of the compound Ia as a raw product.

## Claims

1. Process for the preparation of a compound of formula **I,** or a tautomeric form thereof, or a pharmaceutically acceptable salt thereof, or a pharmaceutically acceptable solvate thereof, including a hydrate, 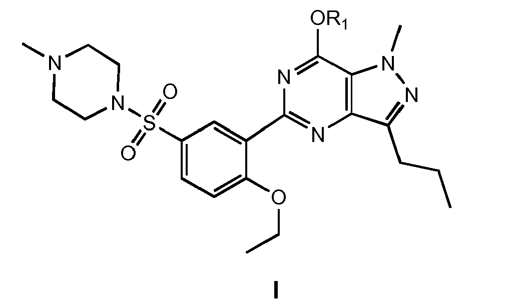 wherein R₁ is selected from H and (C₁-C₄)alkyl; which comprises the coupling reaction of a compound of formula **II** 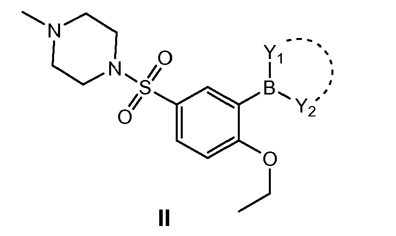 with a compound of formula **III** 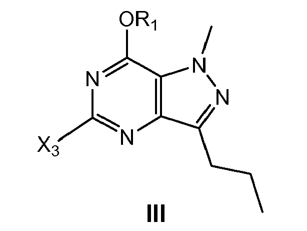 in a suitable solvent in the presence of a metal compound and of a base, wherein:
Y₁ and Y₂ are selected from hydroxyl, (C₁-C₄)alcoxyl and phenoxyl optionally substituted with one or two radicals independently selected from (C₁-C₄)alkyl and (C₁-C₄)alcoxyl; or alternatively Y₁ and Y₂ together with the boron atom can form one of the following cyclic systems: 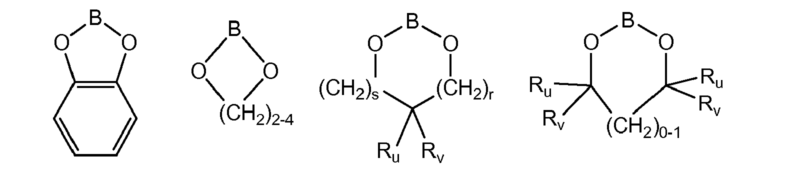 wherein r and s represent independently a value between 0 and 4, provided that r and s may not be concurrently 0; and Rᵤ and Rᵥ are independently selected from H, (C₁-C₄)alkyl and phenyl optionally substituted with one or two radicals independently selected from (C₁-C₄)alkyl and (C₁-C₄)alcoxyl;
X₃ is a leaving group; and
R₁ has the same meaning as in the compound of formula **I;**
and, optionally, the compound of formula **I** wherein R₁ is (C₁-C₄)alkyl is subjected to a hydrolysis reaction to give the compound of formula **I** wherein R₁ is H; and, optionally, the compound of formula **I** is converted into a pharmaceutically acceptable salt thereof by treatment with the corresponding acid, or a salt of the compound of formula **I** may be converted into another salt of the compound of formula **I** by ion exchange,
or the compound of formula **I** is converted into a pharmaceutically acceptable solvate thereof, including a hydrate, by crystallization/precipitation in a suitable solvents system.

2. Process for preparation according to claim 1, wherein Y₁ and Y₂ are selected from hydroxyl, (C₁-C₄)alcoxyl and phenoxyl optionally substituted with one or two radicals independently selected from (C₁-C₄)alkyl and (C₁-C₄)alcoxyl.

3. Process for preparation according to claims 1-2, wherein Y₁ and Y₂ represent hydroxyl.

4. Process for preparation according to any of the claims 1-3, wherein X₃ is selected from Cl, Br, I and trifluoromethanesulfonyloxyl.

5. Process for preparation according to claim 4, wherein X₃ is Cl.

6. Process for preparation according to any of the claims 1-5, wherein the starting product is a compound of formula **III** wherein R₁ is (C₁-C₄)alkyl, and the obtained compound **I** is subjected to a hydrolysis reaction to give Sildenafil.

7. Process for preparation according to claim 6, wherein the starting point is a compound of formula **III** wherein R₁ is methyl or ethyl.

8. Process for preparation according to any of the claims 1 to 5, wherein the starting product is compound of formula **III** wherein R₁ is H so that the compound **I** obtained is Sildenafil.

9. Process for preparation according to any of the claims 1-8, wherein the metal compound is selected from palladium, nickel, a metal salt and a metal complex.

10. Process for preparation according to claim 9, wherein the metal compound is selected from Pd(PPh₃)₄ and Pd(OAc)₂/PPh₃.

11. Compound of formula **I,** or a pharmaceutically acceptable salt thereof, or a pharmaceutically acceptable solvate thereof, including a hydrate, 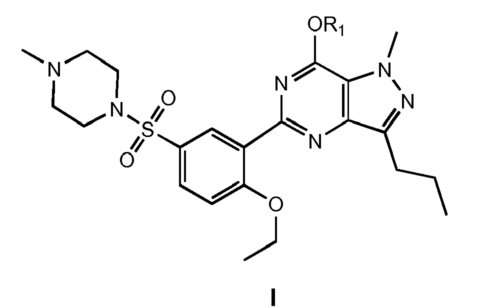 wherein R₁ represents (C₂-C₄)alkyl.

12. 7-ethoxy-5-[2-ethoxy-5-(4-methylpiperazin-1-ylsulfonyl)phenyl]-1-methyl-3-propyl-1*H*-pyrazolo[4,3-*d*]pyrimidine.

13. Compound of formula **II** 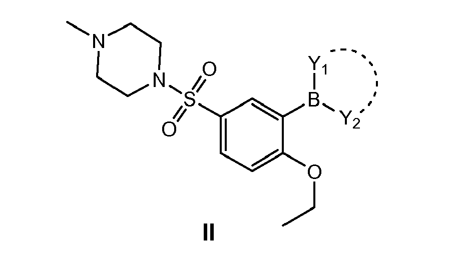 wherein Y₁ and Y₂ have the meaning described in claim 1.

14. Compound according to claim 13, wherein Y₁ and Y₂ are selected from hydroxyl, (C₁-C₄)alcoxyl and phenoxyl optionally substituted with one or two radicals independently selected from (C₁-C₄)alkyl and (C₁-C₄)alcoxyl.

15. Compound according to claims 13 to14, wherein Y₁ and Y₂ represent hydroxyl.

16. [2-Ethoxy-5-(4-methylpiperazin-1-ylsulfonyl)]phenylboronic acid.

17. Process for preparation of a compound of formula **II,** as defined in claim 13, which comprises the reaction of a compound of formula **IV,** 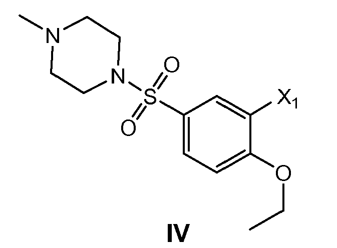 with a metal compound in a suitable solvent, and subsequently with a boronic derivative of formula **XI,** 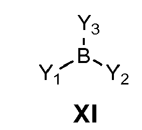 wherein X₁ is a leaving group; and Y₁, Y₂ and Y₃ represent (C₁-C₄)alcoxyl;
and, optionally, the compound of formula **II** obtained, wherein Y₁ and Y₂ represent hydroxyl, may be converted into another compound of formula **II** wherein Y₁ and Y₂ are selected from (C₁-C₄)alcoxyl and phenoxyl optionally substituted with one or two radicals independently selected from (C₁-C₄)alkyl and (C₁-C₄)alcoxyl;
by reaction with an alcohol of formula R₂OH in a suitable solvent, wherein R₂ is selected from (C₁-C₄)alkyl and phenyl optionally substituted with one or two radicals independently selected from (C₁-C₄)alkyl and (C₁-C₄)alcoxyl;
or optionally, the compound of formula **II** obtained, wherein Y₁ and Y₂ represent hydroxyl, may be converted into another compound of formula **II** wherein Y₁ and Y₂ together with the boron atom can form one of the following cyclic systems: 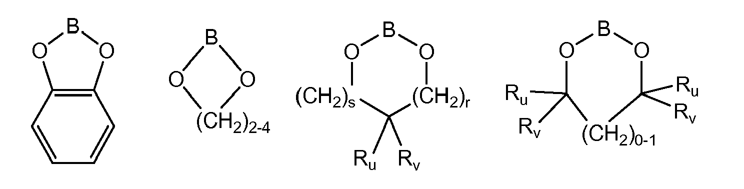 by reaction with a diol of formula: 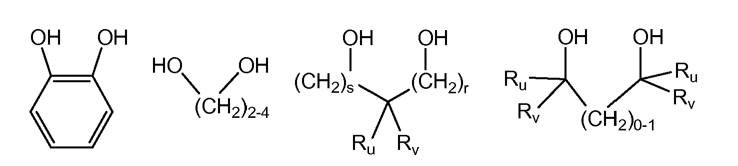 in a suitable solvent, wherein r, s, Rᵤ and Rᵥ have the meaning described in claim 1.
